Europäisches Patentamt

European Patent Office

Office européen des brevets

(11) Numéro de publication: **0 360 653**
**A1**

## (12) DEMANDE DE BREVET EUROPEEN

(21) Numéro de dépôt: **89402440.5**

(22) Date de dépôt: **07.09.89**

(51) Int. Cl.⁵: **A 61 B 6/03**
**G 06 F 15/68**

(30) Priorité: **16.09.88 FR 8812120**

(43) Date de publication de la demande:
**28.03.90 Bulletin 90/13**

(84) Etats contractants désignés: **DE ES GB IT NL**

(71) Demandeur: **GENERAL ELECTRIC CGR S.A.**
**100, rue Camille-Desmoulins**
**F-92130 Issy les Moulineaux (FR)**

(72) Inventeur: **Feldman, Andréi CABINET BALLOT-SCHMIT**
**7, rue Le Sueur**
**F-75116 Paris (FR)**

**Cornuejols, Dominique CABINET BALLOT-SCHMIT**
**7, rue Le Sueur**
**F-75116 Paris (FR)**

(74) Mandataire: **Ballot, Paul Denis Jacques**
**Cabinet Ballot-Schmit 7, rue le Sueur**
**F-75116 Paris (FR)**

(54) **Procédé et système de correction des défauts d'images d'un scanner dus aux déplacements de ce dernier.**

(57) Procédé de correction dans un scanner des défauts dus aux déplacements du scanner qui consiste à disposer un barreau (P) qui introduit un affaiblissement important et faire effectuer au scanner un tour complet autour d'un centre de rotation O et obtenir m vues distinctes du barreau (P) correspondant chacune à une position angulaire $\alpha_j$. L'analyse des signaux de chaque vue permet de déterminer l'angle $\beta_j$ du barreau, la connaissance de cet angle $\beta_j$ conduisant au calcul des coordonnées b et c du barreau P et de la valeur théorique $\beta_{th}$ de l'angle $\beta$ pour chaque vue. La différence entre la valeur théorique $\beta_{th}$ et la valeur mesurée $\beta_j$, est une valeur de correction qui est utilisée dans le dispositif d'élaboration de l'image scanner.

FIG_2

EP 0 360 653 A1

Bundesdruckerei Berlin

## Description

## PROCEDE ET SYSTEME DE CORRECTION DES DEFAUTS D'IMAGES D'UN SCANNER DUS AUX DEPLACEMENTS DE CE DERNIER

L'invention concerne les scanners à rayons X et plus particulièrement un procédé qui permet de corriger, dans les images obtenues, les défauts dus aux déplacements de l'ensemble de mesure; elle concerne également un système de correction de tels défauts.

Pour examiner un patient, on utilise de plus en plus des appareils à rayons X appelés "scanners" qui réalisent des images de coupes transversales du patient. Ces appareils sont basés sur le phénomène physique d'absorption des rayons X par le corps humain. Cette absorption est directement liée à la distance parcourue x des rayons X dans le corps selon la formule :

$$I = I_0 e^{-bx}$$

formule dans laquelle :

$I_0$ est l'intensité du rayonnement entrant dans le corps humain

$I$ est l'intensité du rayonnement sortant du corps humain,

$b$ est un coefficient d'atténuation qui dépend du corps traversé.

Dans une échelle de mesure logarithmique, l'atténuation $I/I_0$ est égale à bx, c'est-à-dire qu'elle est proportionnelle à la distance x.

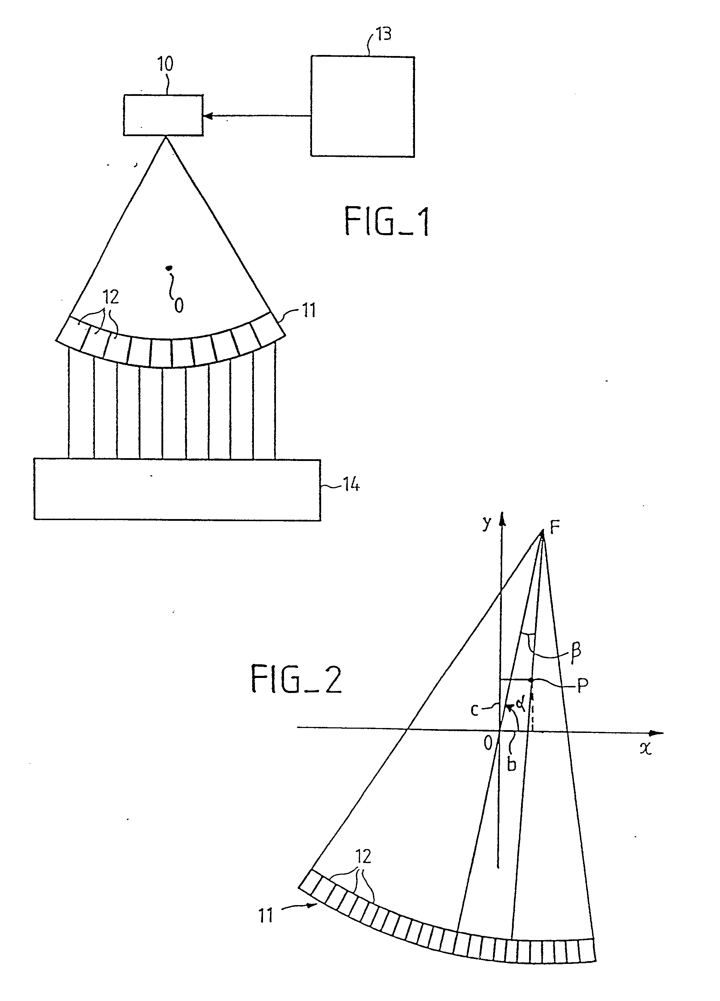

Ces appareils sont constitués essentiellement, comme le montre la figure 1, d'une source 10 de rayons X associée à un dispositif de détection 11, ces deux éléments étant disposés l'un par rapport à l'autre dans une relation géométrique fixe de manière à pouvoir intercaler entre eux le corps à examiner. En outre, ils sont supportés par une structure (non représentée) qui peut tourner autour du corps à examiner de manière à irradier le corps suivant des angles différents. La source de rayons X, qui est commandée par un dispositif 13, émet ses rayons suivant un secteur angulaire qui a une largeur suffisante pour illuminer toute la section transversale du corps. Le dispositif de détection 11 a la forme d'un secteur annulaire dont la longueur est adaptée à la largeur du faisceau de rayons X et est constitué d'un grand nombre de détecteurs élémentaires 12 juxtaposés les uns à côté des autres.

Pour obtenir une image de la section transversale du corps humain traversé par le faisceau de rayons X, on fait tourner la structure de support de la source 10 et du dispositif de détection 11 autour du corps et on mesure les signaux de sortie des détecteurs élémentaires 12 pour les traiter de manière appropriée selon des procédés connus afin d'en tirer une image représentative de la section transversale. Pour ce traitement, les détecteurs élémentaires 12, également appelés canaux, sont connectés à un dispositif électronique 14 qui effectue en premier lieu un calcul du logarithme des signaux reçus de manière à obtenir un signal dont l'amplitude est proportionnelle à l'atténuation des rayons X.

L'axe de rotation de la structure - source 10, dispositif de détection 11 - est matérialisé par le point 0, ce qui signifie que la source 10 et les détecteurs 12 décrivent chacun une circonférence de centre 0 et de rayon déterminé lors de la rotation de la structure. Ceci n'est vrai qu'en théorie car les tolérances de construction et d'utilisation conduisent à des écarts par rapport aux positions théoriques. Il en résulte alors des défauts dans les images appelés "artéfacts" et une diminution de la résolution spatiale.

Le but de la présente invention est donc de mettre en oeuvre un procédé qui permet de corriger les défauts résultant des écarts de position de la source et des détecteurs par rapport à leur position théorique.

Un autre but de l'invention est également de réaliser un système qui met en oeuvre ledit procédé de correction.

L'invention se rapporte à un procédé de correction des défauts d'images d'un scanner dus aux déplacements de ce dernier, caractérisé en ce qu'il comprend les opérations suivantes :

- La mise en place d'au moins un barreau qui introduit un affaiblissement important du rayonnement X incident,

- la rotation du scanner sur un tour complet de manière à réaliser un nombre m de vues sur les N détecteurs ou canaux du scanner, chaque vue correspondant à une position angulaire déterminée $\alpha_j$ du scanner autour de son centre de rotation,

- la détermination, pour chaque vue, du centre de gravité de l'atténuation (barreau) de manière à mesurer un angle $\beta_j$ entre les axes de rayonnement passant l'un par le centre de rotation et l'autre par le centre de gravité de l'atténuation,

- la mesure des coordonnées cartésiennes b et c du centre de gravité de l'atténuation, c'est-à-dire du barreau,

- le calcul pour chaque vue de l'angle théorique $\beta_{th}$ entre les axes de rayonnement passant l'un par le centre de rotation du scanner et l'autre par le point de coordonnées b et c,

- le calcul pour chaque vue de la différence $\delta_j$ entre les angles $\beta_{th}$ et $\beta_j$ et la mise en mémoire des m valeurs $\delta_j$, chaque valeur $\delta_j$ étant ensuite utilisée pour modifier la valeur correspondante de la position angulaire $\alpha_j$.

L'invention se rapporte également à un système pour mettre en oeuvre le procédé décrit ci-dessus dans un scanner comprenant une source de rayonnement X, un dispositif de détection du rayonnement X à N détecteurs, des moyens pour faire tourner, autour d'un centre de rotation, la source de rayonnement X et le dispositif de détection, des moyens pour introduire au moins un barreau entre la source de rayonnement X et le dispositif de détection de rayonnement X, des moyens pour enregistrer dans une mémoire les signaux reçus correspondant à m positions angulaires différentes $\alpha_j$ de la source et du dispositif de détection lors d'un tour complet, caractérisé en ce qu'il comprend en outre :

- des premiers moyens associés à la mémoire pour analyser les signaux contenus dans ladite mémoire de manière à déterminer pour chaque vue la valeur de l'angle $\beta_j$,
- des deuxièmes moyens associés à la mémoire pour analyser les signaux contenus dans ladite mémoire de manière à déterminer les coordonnées b, c du barreau,
- des troisièmes moyens pour calculer l'angle théorique $\beta_{th}$ pour chacune des m positions angulaires $\alpha_j$.
- des quatrièmes moyens pour calculer pour chacune des m vues, la différence angulaire $\beta_{th} - \beta_j = \delta_j$, et
- des cinquièmes moyens pour enregistrer les m valeurs angulaires de correction $\delta_j$.

D'autres caractéristiques et avantages de la présente invention apparaîtront à la lecture de la description suivante d'un exemple particulier de réalisation, ladite description étant faite avec les dessins joints dans lesquels :

- la figure 1 est un schéma de principe d'un scanner à rayons X ;
- la figure 2 est un schéma géométrique qui permet de comprendre le procédé mis en oeuvre; et
- la figure 3 est un schéma fonctionnel d'un système de traitement des signaux des détecteurs mettant en oeuvre le procédé de correction des images d'un scanner selon l'invention.

Le schéma géométrique de la figure 2 permet de mieux comprendre le procédé et de déterminer les corrections à effectuer. Sur cette figure, le point O désigne l'axe de rotation du scanner et les axes Ox et Oy sont deux axes orthogonaux entre eux et à l'axe de rotation, l'un Ox étant horizontal, l'autre Oy étant vertical. La position angulaire de la structure est définie par l'ange $\alpha$ que fait la droite FO avec Ox, F matérialisant le foyer de la source de rayons X. Le point P indique l'emplacement d'un barreau qui absorbe fortement le rayonnement X et dont les coordonnées sont par exemple b pour l'abscisse et c pour l'ordonnée.

Par ailleurs, on appellera $\beta$ l'angle OFP; cet angle $\beta$ varie au cours d'un cycle de rotation du scanner. Cet angle peut aussi se définir comme étant l'angle entre le détecteur central (aligné avec FO) du dispositif de détection 11 et le détecteur de rang i dont le signal est atténué par le barreau P. Ainsi, pour chaque vue d'ordre j, plusieurs canaux présenteront un signal fortement atténué par le barreau P, ce qui permettra de définir un angle $\beta_j$. Selon l'invention, cet angle $\beta_j$ sera comparé par soustraction à l'angle théorique $\beta_{th}$ défini par la relation trigonométrique :

$$\beta_{th} = \text{arctg}\ \frac{\text{OF sin } \alpha_j - c}{\text{OF cos } \alpha_j - b}\ -\ \alpha_j \qquad (1)$$

C'est le résultat de cette soustraction $\beta_{th} - \beta_j$ qui sera utilisé pour effectuer des corrections sur les positions angulaires $\alpha_j$.

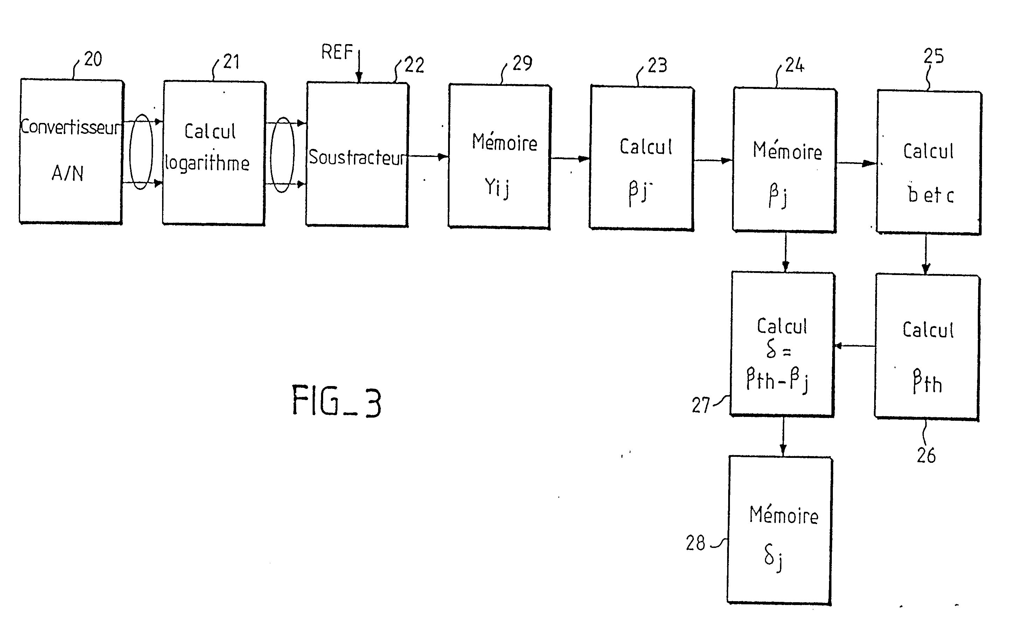

Pour connaître $\beta_{th}$, il faut d'abord déterminer les coordonnées b et c du barreau P, ce qui peut être obtenu de différentes manières, par exemple en effectuant une acquisition sur le barreau conduisant à mesurer b et c sur l'image obtenue. Une autre manière de calculer b et c sera expliquée ci-après en relation avec la description de la figure 3. L'angle $\beta_{th}$ est alors ensuite calculé par la formule (1).

Pour chaque vue j, l'angle $\beta_j$ est déterminé par la position du canal i qui présente le signal le plus atténué, celui correspondant aux rayons X rencontrant le barreau P.

Le procédé de correction des défauts d'images d'un scanner dus aux déplacements de ce dernier comprend donc les opérations suivantes :
- La mise en place au moins d'un barreau introduisant un affaiblissement important du rayonnement X incident,
- la rotation du scanner sur un tour complet de manière à réaliser un nombre m de vues sur les N détecteurs ou canaux, chaque vue correspond à une position angulaire déterminée $\alpha_j$ du scanner autour de son centre de rotation,
- la détermination, pour chaque vue d'ordre j (angle $\alpha_j$), du centre de gravité de l'atténuation (correspondant au barreau P) et ainsi obtenir la valeur de l'angle $\beta_j$,
- la mesure des coordonnées b et c du barreau P,
- le calcul pour chaque vue j de l'angle théorique $\beta_{th}$ à partir des coordonnées mesurées b et c du barreau en utilisant la formule (1);
- le calcul pour chaque vue j de la différence $\delta_j = (\beta_{th} - \beta_j)$ qui constitue une valeur de correction et la mise en mémoire des m valeurs $\delta_j$;
- l'utilisation de chaque valeur $\delta_j$ pour modifier la valeur correspondante des positions angulaires des N détecteurs pour la vue d'ordre j.

Pour mettre en oeuvre ce procédé de correction, l'invention propose un système qui sera maintenant décrit en relation avec la figure 3. Il comprend un convertisseur analogique/numérique 20 auquel sont appliqués les signaux de sortie des N détecteurs ou canaux du dispositif de détection 11. Les N codes numériques correspondant à une position ou vue de rang j sont appliqués à un circuit de calcul de logarithme 21 qui fournit, pour chaque canal i et chaque vue j un code représentatif de l'atténuation subie par le rayonnement X. Les N codes résultant de cette opération de calcul logarithmique sont appliqués à un circuit de soustraction 22 dans lequel on leur soustrait une valeur REF représentative de l'atténuation subie par le rayonnement X dans l'air.

Cette valeur est obtenue à l'aide d'un détecteur appelé moniteur dont la position sur le dispositif de détection 11 est telle qu'il reçoit le rayonnement X sans atténuation.

Les codes qui résultent de cette soustraction, appelés mesures $Y_{ij}$, sont enregistrées dans une mémoire 29 puis traitées dans un dispositif de calcul 23 afin de déterminer pour chaque vue j, l'angle $\beta_j$ correspondant au barreau P. Ceci est par exemple effectué en analysant les N signaux $Y_i$ de chaque vue pour déterminer le canal i qui représente le centre gravité de l'atténuation due au barreau P. Plus précisément, on détermine les canaux qui correspondent à des signaux atténués par le barreau P, atténuation dont le maximum ne correspond pas forcément à la position angulaire exacte d'un canal. En d'autres mots, la droite FP n'est pas en général alignée avec le centre d'un détecteur 12 (Figure 2). Aussi, il est nécessaire de déterminer le maximum de l'atténuation et on obtient alors une valeur de i qui n'est pas entière mais fractionnaire. L'angle $\beta_j$ est alors calculé par le nombre de canaux ou de détecteurs 12 qui sont entre le détecteur central, aligné avec FO, et le détecteur de rang i (i fractionnaire) correspondant à l'atténuation maximale, en tenant compte du fait que les détecteurs sont régulièrement disposés sur un arc de cercle de centre F.

Les m valeurs $\beta_j$ sont enregistrées dans une mémoire 24 afin de servir, d'une part, de base de calcul des coordonnées b et c et, d'autre part, pour obtenir les valeurs $\delta_j$ de correction.

Le calcul de b et c est effectué dans un circuit 25 en utilisant la formule (1) dans laquelle $\beta_{th}$ est remplacé par $\beta_j$ mesuré. Plus précisément, on utilise les valeurs successives de $\beta_j$ deux à deux de manière à obtenir à chaque fois un système de deux équations à deux inconnues b et c dans lequel $\beta_j$ et $\alpha_j$ sont connus. Chaque système d'équations est du type :

$$\beta_j = \text{arctg}\ \frac{OF \sin \alpha_j - c_j}{OF \cos \alpha_j - b_j}\ -\ \alpha_j$$

$$\beta_{j+1} = \text{arctg}\ \frac{OF \sin \alpha_{j+1} - c_j}{OF \cos \alpha_{j+1} - b_j}\ -\ \alpha_{j+1}$$

avec j variant de 1 à m de manière à obtenir m/2 systèmes d'équations. On comprendra que $\beta_{m+1}$ correspond en fait à $\beta_1$.

La résolution des m/2 systèmes aboutit à m/2 valeurs de b et m/2 valeurs de c dont on calcule les moyennes.

Ce sont ces valeurs moyennes de b et c qui sont transmises à un circuit 26 qui réalise pour chaque vue de position $\alpha_j$ le calcul des m valeurs théoriques $\beta_{th}$.

Chacune des m valeurs $\beta_j$ contenues dans la mémoire 24 est soustraite algébriquement dans un circuit 27 à la valeur correspondante (même angle $\alpha_j$ c'est-à-dire même vue) qui est fournie par le circuit de calcul 26. On obtient alors m valeurs différentielles $\delta_j$ qui sont enregistrées dans une mémoire 28 pour être utilisées dans le dispositif d'élaboration de l'image en modifiant les angles $\alpha_{ij}$ qui indiquent les positions angulaires des détecteurs par rapport à l'axe Ox.

L'invention a été décrite en utilisant un seul barreau mais elle peut être aussi mise en oeuvre en utilisant plusieurs barreaux à différentes positions.

Le système qui a été décrit en relation avec la figure 3 peut être réalisé suivant différentes variantes sans sortir du cadre de la présente invention. Notamment, le mémoire 29, qui enregistre les valeurs $Y_{ij}$, peut être supprimée si le calcul de $\beta_j$ (circuit 23) est effectué en temps réel.

De même, le calcul des coordonnées b et c du barreau P peut être réalisé de différentes manières, par exemple sur l'image du barreau obtenu par le scanner comme on l'a signalé ci-dessus.

## Revendications

1. Procédé de correction des défauts d'images d'un scanner dus aux déplacements de ce dernier, caractérisé en ce qu'il comprend les opérations suivantes :
- La mise en place d'au moins un barreau (P) qui introduit un affaiblissement important du rayonnement X incident,
- la rotation du scanner sur un tour complet de manière à réaliser un nombre m de vues sur les N détecteurs ou canaux du scanner, chaque vue correspondant à une position angulaire déterminée $\alpha_j$ du scanner autour de son centre de rotation,
- la détermination, pour chaque vue, du centre de gravité de l'atténuation de manière à mesurer un angle $\beta_j$ entre les axes de rayonnement passant l'un par le centre de rotation et l'autre par le détecteur présentant le centre de gravité de l'atténuation.
- la mesure des coordonnées cartésiennes b et c du centre de gravité du barreau (P),

4

- le calcul pour chaque vue de l'angle théorique $\beta_{th}$ entre les axes de rayonnement passant l'un par le centre de rotation du scanner et l'autre par le point de coordonnées b et c,
- le calcul pour chaque vue de la différence $\delta_j$ entre les angles $\beta_{th}$ et $\beta_j$ et la mise en mémoire des m valeurs $\delta_j$, chacune de ces m valeurs étant ensuite utilisées pour modifier la valeur correspondante de la position angulaire $\alpha_j$.

2. Procédé selon la revendication caractérisé en ce que l'opération de détermination de l'angle $\beta_j$ consiste à analyser, pour chaque vue, les N signaux reçus de manière à déterminer la position angulaire du centre de gravité du signal le plus faible et à la comparer à la position angulaire correspondant à l'alignement foyer (F) du rayonnement X et du centre O de rotation du scanner.

3. Procédé selon la revendication 1 ou 2, caractérisé en ce que l'opération de mesure des coordonnées cartésiennes b et c du barreau (P) est réalisée à partir des m valeurs $\beta_j$ en résolvant m/2 systèmes de deux équations à deux inconnues du type :

$$\beta_j = \text{arctg} \frac{OF \sin \alpha_j - c_j}{OF \cos \alpha_j - b_j} - \alpha_j$$

$$\beta_{j+1} = \text{arctg} \frac{OF \sin \alpha_{j+1} - c_j}{OF \cos \alpha_{j+1} - b_j} - \alpha_{j+1}$$

(j variant de 1 à m) et en calculant les moyennes des m/2 valeurs obtenues pour b et c.

4. Procédé selon la revendication 1, 2 ou 3, caractérisé en ce que l'opération de calcul pour chaque vue de l'angle théorique $\beta_{th}$ est effectuée selon la formule :

$$\beta_{th} = \text{arctg} \frac{OF \sin \alpha_j - c}{OF \cos \alpha_j - b} - \alpha_j$$

5. Système pour mettre en oeuvre le procédé selon l'une quelconque des revendications précédentes dans un scanner comprenant une source (10) de rayonnement X, un dispositif de détection (11) du rayonnement X à N détecteurs, des moyens pour faire tourner, autour d'un centre de rotation, la source de rayonnement X et le dispositif de détection, des moyens pour enregistrer dans une mémoire les signaux reçus correspondant à m positions angulaires différentes $\alpha_j$ de la source et du dispositif de détection lors d'un tour complet, caractérisé en ce qu'il comprend en outre :
- des premiers moyens (23) associés à la première mémoire pour analyser les signaux contenus dans ladite mémoire de manière à déterminer pour chaque vue la valeur de l'angle $\beta_j$,
- des deuxièmes moyens (25) pour déterminer les coordonnées b, c du barreau (P),
- des troisièmes moyens (26) pour calculer l'angle théorique $\beta_{th}$ pour chacune des m positions angulaires $\alpha_j$.
- des quatrièmes moyens (27) pour calculer pour chacune des m vues, la différence angulaire $\beta_{th} - \beta_j = \delta_j$, et
- des cinquièmes moyens (28) pour enregistrer les m valeurs angulaires de correction $\delta_j$.

FIG_1

FIG_2

FIG_3

EP 0 360 653 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-A-2 397 179  (EMI LTD) <br> * Page 4, ligne 20 - page 5, ligne 5; page 8, ligne 2 - page 9, ligne 27; figures 1,2 * <br> --- | 1,5 | A 61 B    6/03 <br> G 06 F   15/68 |
| A | US-A-4 068 306  (A.C.M. CHEN et al.) <br> * Résumé; colonne 2, lignes 8-15; colonne 3, lignes 35-49; colonne 4, lignes 3-32; colonne 12, ligne 4 - colonne 13, ligne 15; figures 1,2,6 * <br> --- | 1,5 | |
| A | EP-A-0 218 367  (PICKER INT. INC.) <br> * Résumé; colonne 5, ligne 24 - colonne 7, ligne 9; figures 1,2 * <br> ----- | 1,5 | |

**DOMAINES TECHNIQUES RECHERCHES (Int. Cl.5)**

A 61 B
H 05 G

Le présent rapport a été établi pour toutes les revendications

| Lieu de la recherche | Date d'achèvement de la recherche | Examinateur |
|---|---|---|
| LA HAYE | 22-12-1989 | RIEB K.D. |